# EUROPÄISCHE PATENTANMELDUNG

(11) **EP 1 125 926 A1**
(43) Veröffentlichungstag der Anmeldung: **22.08.2001**
(21) Anmeldenummer: 01102381.9
(22) Anmeldetag: 02.02.2001
(51) Int. Cl.: C07D 211/82, C07D 213/55

(54) **Verfahren zur Herstellung von 4-(4'-Carboxyphenyl)pyridin**

(30) Priorität: 15.02.2000 DE 10006601
(71) Anmelder: Clariant GmbH, 65929 Frankfurt am Main (DE)
(72) Erfinder: Meudt, Andreas, Dr., 65439 Flörsheim-Weilbach (DE); Scherer, Stefan, Dr., 64572 Büttelborn (DE); Koch, Peter, Dr., 60318 Frankfurt am Main (DE); Demmer, Gerhard, 65779 Kelkheim (DE); Vollmüller, Frank, Dr., 60323 Frankfurt am Main (DE)

(57) **Zusammenfassung**

Die Erfindung betrifft ein Verfahren zur Herstellung von 4-(4'-Carboxyphenyl)pyridin, dadurch gekennzeichnet, dass man ein 4-Phenyl-N-acyl-dihydropyridin der Formel (II) 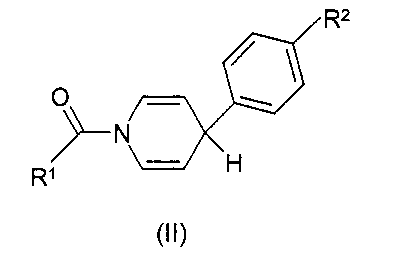 worin
- R¹: eine sterisch anspruchsvolle Alkyl-, Alkylaryl-, Arylalkyl- oder Alkoxygruppe und
- R²: ein geradkettiger oder verzweigter, substituierter oder unsubstituierter Alkylrest mit 1 bis 8 C-Atomen,
bedeuten,
mit einem Oxidationsmittel aus der Gruppe der Permanganate, Salpetersäure, Cr(VI)-Verbindungen, Sauerstoff und Luft zur Verbindung der Formel (I) oxidiert 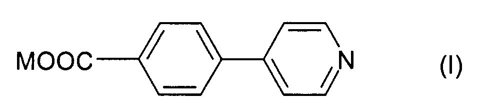 wobei M ein Kation ist.

## Beschreibung

Die Erfindung betrifft ein neues Verfahren zur Herstellung von 4-(4'-Carboxyphenyl)pyridin der Formel (I). 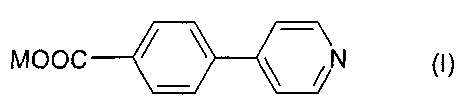 4-(4'-Carboxyphenyl)pyridin der Formel (I) und die entsprechenden Ester und Säurechloride sind bedeutsame Zwischenprodukte in der Synthese von Wirkstoffen der pharmazeutischen und agrochemischen Industrie.

Mögliche synthetische Zugänge zu Verbindungen der Formel (I) bestehen in der Suzuki-Kupplung von 4-Halogenpyridinen mit geeigneten metallorganischen Reagenzien, beispielsweise 4-Carboxyphenylboronsäure oder 4-Carboxyphenylboronsäureestern. Solche Pyridine, wie beispielsweise 4-Brompyridin oder 4-Chlorpyridin, sind aber zum einen sehr teure und schwierig zugängliche Rohstoffe, die in reiner Form zudem instabil sind und nur in derivatisierter Form, beispielsweise als Hydrochlorid, verwendet werden können. Zum anderen sind sie stark korrosiv und stellen entsprechend hohe Anforderungen an die verwendeten Werkstoffe.

Der vorliegenden Erfindung lag die Aufgabe zugrunde, ein Verfahren zur Herstellung von 4-(4'-Carboxyphenyl)pyridin der Formel (I) zu entwickeln, das die Zielverbindung in möglichst wenigen, verfahrenstechnisch einfachen Stufen und ausgehend von gut zugänglichen und kostengünstigen Rohstoffen erhältlich macht. Des weiteren muss das zu entwickelnde Verfahren das Zielprodukt in einer guten Gesamtausbeute und in einer für pharmazeutische Anwendungen ausreichenden Reinheit liefern.

Überraschenderweise wurde gefunden, dass sich die Verbindung der Formel (I) in wenigen Stufen, mit guten Gesamtausbeuten und in hoher Reinheit, ausgehend von Pyridin, herstellen lässt. Die einzelnen Stufen sind dabei jeweils verfahrenstechnisch einfach durchführbar und verwenden ausschließlich kommerziell erhältliche und kostengünstige Reagenzien, Lösungsmittel und Katalysatoren.

Gegenstand der vorliegenden Erfindung ist ein Verfahren zur Herstellung von 4-(4'-Carboxyphenyl)pyridin, dadurch gekennzeichnet, dass man ein 4-Phenyl-N-acyl-dihydropyridin der Formel (II) 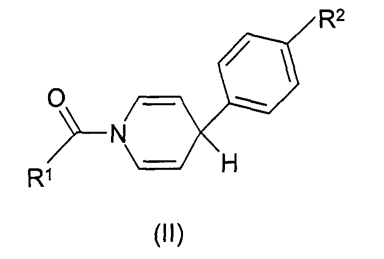 worin
- R¹: eine sterisch anspruchsvolle Alkyl-, Alkylaryl-, Arylalkyl- oder Alkoxygruppe und
- R²: ein geradkettiger oder verzweigter, substituierter oder unsubstituierter Alkylrest mit 1 bis 8 C-Atomen,
bedeuten,
mit einem Oxidationsmittel aus der Gruppe der Permanganate, Salpetersäure, Cr(VI)-Verbindungen, Sauerstoff und Luft zur Verbindung der Formel (l) oxidiert 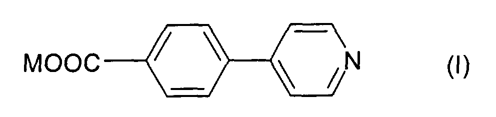 wobei M ein Kation ist, vorzugsweise Wasserstoff, Ammonium oder ein Alkalimetallkation.

Bevorzugte Reste R¹ sind tert.-Butyl, Isopropyl, (Dimethyl)phenylmethyl, Methyl(diphenyl)-methyl, Trityl, Diphenylmethyl, Triethylmethyl, tert.-Butoxy und iso-Propoxy.

Bevorzugte Reste R² sind geradkettige oder verzweigte, unsubstituierte oder mit ein oder mehreren Hydroxy- oder C₁-C₄-Alkoxyresten substituierte Alkylreste mit 1 bis 4 C-Atomen, insbesondere Methyl, Ethyl, n-Propyl, i-Propyl, n-Butyl, Methoxymethyl und Hydroxymethyl, besonders bevorzugt ist Methyl.

Die Herstellung von Dihydropyridinen der Formel (II) aus Pyridin durch Addition metallorganischer Verbindungen, beispielsweise Grignard-Verbindungen, gelingt nicht direkt; eine vorherige Aktivierung des Pyridinrings ist dazu erforderlich. Dies kann beispielsweise durch N-Acylierung erfolgen (Schema 1). Die so erhältlichen N-Acylpyridiniumsalze reagieren dann zwar mit Grignard-Verbindungen, man erhält aber schwer auftrennbare Gemische aus 2- und 4-Aryldihydropyridinen. Hohe Selektivität zu den 4-Aryldihydropyridinen läßt sich nach Akiba et al., Tetrahedron Lett. 23, 4, 429-432, 1982 erreichen durch Einsatz sperriger Reste am Pyridin-N und Einsatz von equimolaren Mengen kupferorganischer Verbindungen. Die Herstellung und Handhabung Kupfer-organischer Verbindungen sind aber aus einer Reihe von Gründen für technische Verfahren als problematisch zu bewerten. 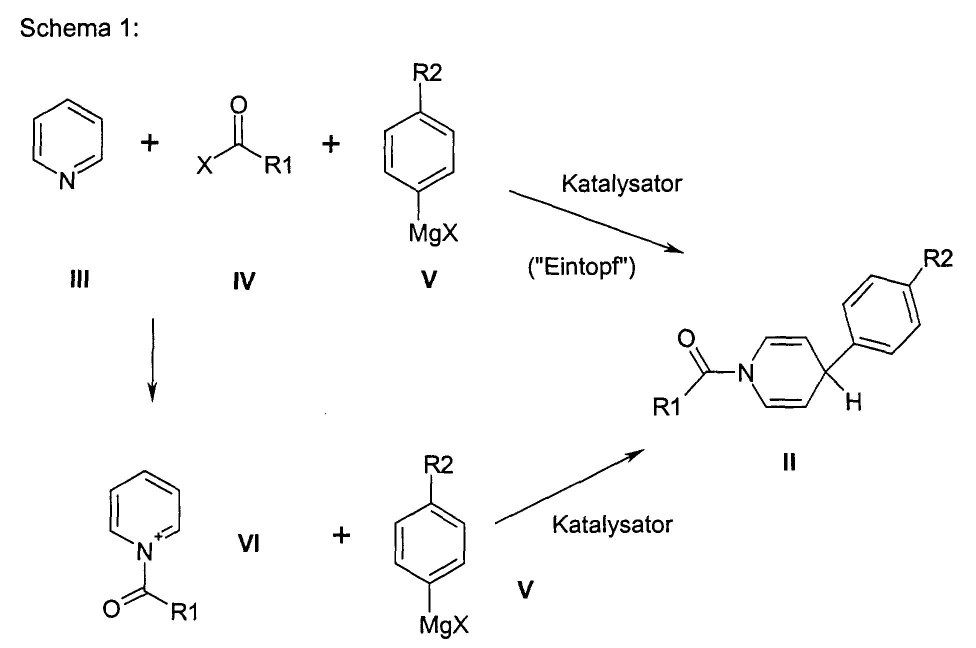 X kann Cl oder Br bedeuten.

Eleganter wird das Problem durch Comins et al., J. Org. Chem. 1982, 47, 4315-4319, gelöst. Die Autoren setzen Grignardverbindungen in Anwesenheit katalytischer Mengen Cul mit Pyridin und Pivaloylchlorid um und erhalten so Ausbeuten von etwas über 60 %. Die hierbei benötigten Cu-Mengen stünden einer technischen Nutzung nicht im Wege. Allerdings stellt eine so niedrige Ausbeute verbunden mit den Entsorgungsproblemen für die teilweise problematischen Nebenprodukte, die aus den nicht zum Produkt umgesetzten Edukten resultieren, die Rentabilität des gesamten Prozesses in Frage.

Überraschenderweise wurde gefunden, dass der Zusatz von Palladium- oder Nickel-Salzen oder -Komplexen oder von metallischem Pd oder Ni als Cokatalysatoren bei der Herstellung eines Dihydropyridins der Formel (II) zu einer deutlichen Ausbeutesteigerung führt. Es werden dann Ausbeuten von meist über 90 %, bezogen auf das eingesetzte metallorganische Reagenz, erreicht.

In einer bevorzugten Ausführungsform des erfindungsgemäßen Verfahrens wird daher die Verbindung der Formel (II) hergestellt, indem man Pyridin mit einer Verbindung der Formel (IV) unter Erhalt einer Verbindung der Formel (VI) acyliert oder eine Verbindung der Formel (VI) einsetzt, und mit einer Grignard-Verbindung der Formel (V) in Gegenwart von 0,01 bis 10 mol%, vorzugsweise 0,1 bis 5 mol%, bezogen auf die Grignard-Verbindung, einer Cu-Verbindung, und eines Pd- oder Ni-Cokatalysators kuppelt, 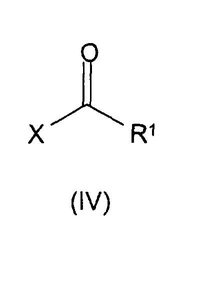 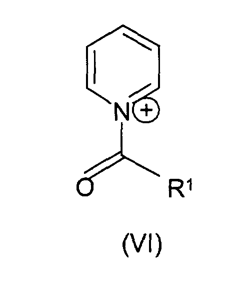 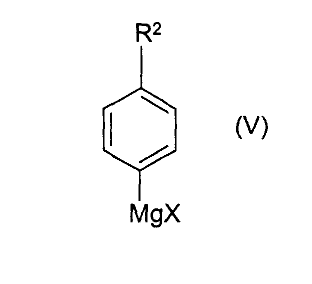 worin X die Bedeutung Cl oder Br hat.

Geeignete Lösungsmittel für die Kupplung der Verbindung der Formel (IV) mit (V) sind beispielsweise aliphatische oder aromatische Ether oder Kohlenwasserstoffe, bevorzugt THF oder THF/Toluol-Gemische. Die Kupplung kann bei Temperaturen zwischen -80°C und dem Siedepunkt des verwendeten Lösungsmittels durchgeführt werden. Bei hohen Temperaturen nimmt aber der Anteil des 2-Substitutionsproduktes auf Kosten des gewünschten 4-Substitutionsproduktes zu, so dass Temperaturen zwischen -70 und + 60°C bevorzugt sind. Besonders bevorzugt sind Temperaturen zwischen -50 und + 50°C, ganz besonders bevorzugt -35 bis + 40°C. Als Cu-Verbindungen kommen Cu(I)- und Cu(II)-Verbindungen in Betracht, bevorzugt Cul, CuBr, CuCl oder CuCl₂.

Geeignete Cokatalysatoren für die Kupplungsreaktionen sind Salze, Komplexe oder die metallische Form von Nickel oder Palladium. Die Anwendung findenden Mengen können zwischen 10⁻⁵ und 10 mol-%, bevorzugt zwischen 10⁻⁴ und 7,5 mol%, insbesondere zwischen 10⁻³ und 5 mol%, bezogen auf die Grignard-Verbindung, liegen.

Besonders bevorzugt sind Salze oder Komplexe von Nickel oder Palladium sowie metallische Formen, gegebenenfalls auf einem geeigneten Träger, z.B. Pd auf C oder auf BaSO₄, ganz besonders bevorzugt PdCl₂(dppf), PdCl₂(PPh₃)₂, PdCl₂(dppe), PdCl₂(dppp), PdCl₂(dppb), Pd(PPh₃)₄, Pd(OAc)₂, PdCl₂, PdBr₂ oder NiCl₂(PPh₃)₂, worin "dppf" 1,2-Bis(diphenylphosphino)ferrocen, "dppe" 1,2-Bis(diphenylphosphino)ethan, "dppp" 1,2-Bis(diphenylphosphino)propan, "dppb" 1,2-Bis-(diphenylphosphino)butan, "Ph" Phenyl und "Ac" Acetyl bedeuten.

Die Umwandlung des Dihydropyridins der Formel (II) in das 4-(4'-Carboxyphenyl)-pyridin der Formel (I) geschieht erfindungsgemäß durch Oxidation mit einem Oxidationsmittel aus der Gruppe der Permanganate, z.B. Na- oder K-Permanganat, Salpetersäure, Chrom(VI)-Reagenzien, z.B. Alkalichromate oder Alkalidichromate, Sauerstoff oder Luft, besonders bevorzugt durch Luftoxidation.

Nach Comins et al. ist es möglich, N-acylierte 4-Aryldihydropyridine durch Erhitzen mit elementarem Schwefel in die entsprechenden 4-Arylpyridine umzuwandeln.

Bei der Behandlung mit Schwefel entstehen jedoch zum einen große Mengen von Schwefelwasserstoff und anderer Schwefelverbindungen, zum anderen sind dabei hohe Temperaturen erforderlich, wodurch ein sehr unreines, schwarzes Produkt anfällt. Dieses lässt sich nur sehr umständlich in eine für weitere Oxidationen erforderlichen Reinheit überführen.

Überraschenderweise wurde gefunden, dass die Umwandlung des Dihydropyridins der Formel (II) durch Oxidation in einer einzigen Stufe und unter Erhalt eines sehr reinen Produkts der Formel (I) möglich ist. Trotz der dabei aufeinanderfolgenden Oxidationen des N-Acylrestes, des Dihydropyridins zum Pyridin und der aromatischen Alkylgruppe zum Säurerest werden dabei gute Ausbeuten eines reinen Produktes erhalten.

Die Oxidation kann mit Permanganaten, Salpetersäure, Luft, O₂, oder Chrom(VI)-Reagenzien durchgeführt werden, als besonders vorteilhaft erwies sich dabei aber die Oxidation mit Luft. Hierbei werden nahezu quantitative Ausbeuten und hohe Reinheit erhalten. Geringe Mengen der einzigen nachweisbaren Verunreinigung, Terephthalsäure, lassen sich durch Waschen mit verdünnten Laugen leicht und quantitativ entfernen.

Die Oxidation mit Permanganat kann sowohl in wässriger Lösung als auch in nichtwässrigem Medium geschehen, bevorzugt bei Tempraturen von 0 bis 100°C, insbesondere 10 bis 80°C. In wässriger Lösung ist die Gegenwart geeigneter Phasentransferkatalysatoren, beispielsweise Tetraalkylammoniumsalzen, Tetraphenylphosphoniumsalzen oder Kronenethern, für die Erzielung guter Ausbeuten förderlich. Die Aufarbeitung geschieht durch Filtration des entstandenen Braunsteins, Ansäuern und Abfiltrieren der ausfallenden Pyridylbenzoesäure.

Die Oxidation mit Luft oder Sauerstoff wird in aliphatischen Carbonsäuren, gegebenenfalls im Gemisch mit Wasser, durchgeführt. Bevorzugt wird Essigsäure verwendet, besonders bevorzugt ist ein Gemisch aus Essigsäure und Wasser. Als Katalysator dienen Schwermetallsalze, z.B. Gemische aus Cobalt- und Manganbromid. Die Schwermetallsalze werden in Mengen von je 0,01 bis 5,0 Mol-%, bevorzugt 0,1 bis 4,0 Mol-%, besonders bevorzugt 1,0 bis 2,0 Mol-%, bezogen auf Dihydropyridin (II) eingesetzt. Das Verhältnis zwischen Kobalt und Mangan lässt sich in weiten Grenzen variieren und kann beispielsweise zwischen 1:5 und 5:1 liegen. Bevorzugt werden die beiden Salze äquimolar eingesetzt. Die Reaktion wird bei Temperaturen zwischen 100 und 200°C, bevorzugt zwischen 120 und 180°C und besonders bevorzugt zwischen 150 und 170°C durchgeführt. Der Druck liegt zwischen Normaldruck und 50 bar. Die Aufarbeitung geschieht durch Abkühlen und gegebenenfalls Einengen der Reaktionsmischung, Filtration, Waschen und Trocknung der entstandenen ausgefallenen Carbonsäure.

### Beispiele

Herstellung von 4-p-Tolyl-N-pivaloyldihydropyridin

### Beispiel 1

Zu einer Lösung von 121 ml Pyridin (1,5 mol), 3,8 g Cul (0,02 mol) und 9 mg PdCl₂(dppf) in 200 ml Tetrahydrofuran werden bei-15°C 1,0 Mol p-Tolylmagnesiumchlorid (25 Gew.-% Lösung in THF) zugetropft. Anschließend werden bei der gleichen Temperatur 123 ml Pivaloylchlorid (1,0 Mol) in 15 Minuten zugetropft. Die Reaktion ist stark exotherm, gute Kühlung ist erforderlich. Nach 15-minütigem Nachrühren bei -15°C wird das Kältebad entfernt und die Reaktionsmischung über Nacht bei Raumtemperatur nachgerührt. Nach Hydrolyse mit 500 ml 20 gew.-%iger wässriger Ammoniumchloridlösung, Extraktion der wässrigen Phase mit Toluol, Trocknen mit Magnesiumsulfat und Abdestillieren des größten Teils der Lösemittel kristallisiert das Dihydropyridin als farbloser Feststoff aus und kann durch Filtration sehr rein gewonnen werden. Die Ausbeute an Dihydropyridin beträgt 364,7 g (1,43 Mol, 95 %).

### Beispiel 2

Zu einer Lösung von 121 ml Pyridin (1,5 mol), 1,5 g (0,0079 mol) Cul und 0,9 mg PdCl₂(dppf) in 200 ml Tetrahydrofuran werden bei -15°C 1,0 Mol p-Tolylmagnesiumchlorid (25 Gew.-% Lösung in THF) zugetropft. Anschließend werden bei der gleichen Temperatur 123 ml Pivaloylchlorid (1,0 Mol) in 15 Minuten zugetropft. Nach der in Beispiel 1 beschriebenen Aufarbeitung erhält man 85 % des Dihydropyridins.

### Beispiel 3 (Vergleichsbeispiel aus D. L. Comins et al., J. Org. Chem. 1982, 47, 4315-4319)

Zu einer Lösung von 12,1 ml Pyridin in 200 ml Tetrahydrofuran werden bei Raumtemperatur 952 mg Cul zugegeben und solange gerührt, bis eine homogene Lösung entstanden ist. Nach dem Abkühlen auf -20°C wird eine Lösung von 0,1 Mol Phenylmagnesiumchlorid in 50 ml THF zugegeben. In 5 Minuten werden 12,3 ml Pivaloylchlorid in 10 ml THF zugetropft. Nach 15-minütigem Nachrühren bei -15°C und weiteren 15 Minuten bei Raumtemperatur wird mit 75 ml 20 gew.-%iger Ammoniumchlorid-Lösung hydrolysiert, mit 200 ml Ether versetzt und die organische Phase nacheinander mit jeweils 50 ml NH₄Cl/NH₄OH 50:50, Wasser, 10 % HCI, Wasser und gesättigter Kochsalzlösung gewaschen. Nach dem Trocknen mit MgSO₄ werden die Lösungsmittel im schwachen Vakuum abdestilliert. Das Produkt verbleibt als gelber Feststoff in einer Ausbeute von 63 %.

### Beispiel 4 Oxidation des Dihydropyridins mit Kaliumpermanganat

Zu 75 ml einer 2 gew.-%igen wässrigen Kaliumpermaganat-Lösung werden bei Raumtemperatur 1,0 g wasserfreies Natriumcarbonat, 0,05 g Benzyltributylammoniumchlorid und 0,8 g 4-p-Tolyl-N-pivaloyldihydropyridin gegeben. Nach 1,5-stündigem Rühren bei 100°C wird nach Reduktion von überschüssigem Permanganat mit Natriumdithionit, Filtration des ausgefallenen Braunsteins, Ansäuern und Filtration des ausgefallenen Produkts 4-(4'-Carboxyphenyl)pyridin in einer Ausbeute von 81 % erhalten.

### Beispiel 5 Oxidation des Dihydropyridins mit Luft

In einem 3,5 l-Autoklaven werden 177,2 g (0,75 Mol) 4-p-Tolyl-N-pivaloyldihydropyridin, 1511,2 g Essigsäure, 3,74 g (15,0 mMol) Cobalt(II)-acetattetrahydrat, 3,68 g (15,0 mMol) Mangan(ll)-acetat-tetrahydrat und 3,09 g (30,0 mMol) Natriumbromid vorgelegt. Der Autoklav wird mit Stickstoff inertisiert und auf 160-165°C geheizt. Bei Erreichen dieser Temperatur werden ca. 450 l/h Luft eingeleitet und über eine Druckhalteeinrichtung ein Innendruck von 16-18 bar aufrecht erhalten. Man leitet ca. 30-40 Minuten Luft ein, inertisiert dann wieder durch Aufdrücken von Stickstoff und kühlt die Mischung ab. Die grünliche Lösung (wird beim Abkühlen orangerot) wird aus dem Autoklaven ausgenommen und am Rotationsverdampfer auf 370 g eingeengt. Die dickflüssige Suspension wird abgenutscht und die Kristalle 3 mal mit je 50 g 75 gew.-%iger Essigsäure gewaschen. Das so erhaltene Rohprodukt wird in 75 ml 0,4 gew.-%iger Natriumbicarbonat-Lösung aufgenommen, bei etwa 50°C 30 Minuten gerührt und abfiltriert. Nach dem Trocknen verbleibt 4-(4'-Carboxyphenyl)pyridin in einer Ausbeute von 90 %. HPLC-Reinheit> 98 % (a/a).

## Patentansprüche

1. Verfahren zur Herstellung von 4-(4'-Carboxyphenyl)pyridin, dadurch gekennzeichnet, dass man ein 4-Phenyl-N-acyl-dihydropyridin der Formel (II) 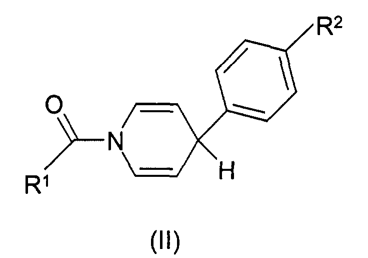 worin
R¹ eine sterisch anspruchsvolle Alkyl-, Alkylaryl-, Arylalkyl- oder Alkoxygruppe und
R² ein geradkettiger oder verzweigter, substituierter oder unsubstituierter Alkylrest mit 1 bis 8 C-Atomen,
bedeuten,
mit einem Oxidationsmittel aus der Gruppe der Permanganate, Salpetersäure, Cr(VI)-Verbindungen, Sauerstoff und Luft zur Verbindung der Formel (I) oxidiert 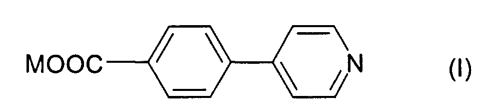 wobei M ein Kation ist.

2. Verfahren nach Anspruch 1, dadurch gekennzeichnet, dass R¹ die Bedeutung tert.-Butyl, Isopropyl, (Dimethyl)phenylmethyl, Methyl(diphenyl)methyl, Trityl, Diphenylmethyl, Triethylmethyl, tert.-Butoxy oder iso-Propoxy hat.

3. Verfahren nach Anspruch 1 oder 2, dadurch gekennzeichnet, dass R² die Bedeutung Methyl, Ethyl, n-Propyl, i-Propyl, n-Butyl, Methoxymethyl oder Hydroxymethyl hat.

4. Verfahren nach mindestens einem der Ansprüche 1 bis 3, dadurch gekennzeichnet, dass das Oxidationsmittel Kaliumpermanganat, Natriumpermanganat, Salpetersäure, ein Alkalichromat, ein Alkalidichromat, O₂ oder Luft ist.

5. Verfahren nach mindestens einem der Ansprüche 1 bis 4, dadurch gekennzeichnet, dass die Oxidation mit Permanganaten in Gegenwart von Phasentransferkatalysatoren, vorzugsweise Tetraalkylammoniumsalzen, Tetraphenylphosphoniumsalzen oder Kronenethern, durchgeführt wird.

6. Verfahren nach mindestens einem der Ansprüche 1 bis 4, dadurch gekennzeichnet, dass die Oxidation mit Luft in Gegenwart von Schwermetallsalzen durchgeführt wird.

7. Verfahren nach Anspruch 6, dadurch gekennzeichnet, dass als Schwermetallsalz ein Gemisch aus Cobalt- und Manganbromid verwendet wird.

8. Verfahren nach mindestens einem der Ansprüche 1 bis 7, dadurch gekennzeichnet, dass die Verbindung der Formel (II) hergestellt wird, indem man Pyridin mit einer Verbindung der Formel (IV) unter Erhalt einer Verbindung der Formel (VI) acyliert oder eine Verbindung der Formel (VI) einsetzt, und mit einer Grignard-Verbindung der Formel (V) in Gegenwart von 0,01 bis 10 mol%, vorzugsweise 0,1 bis 5 mol%, bezogen auf die Grignard-Verbindung, einer Cu-Verbindung, und eines Pd- oder Ni-Cokatalysators kuppelt, 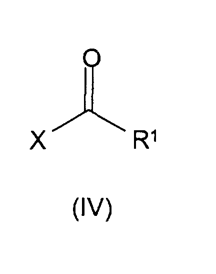 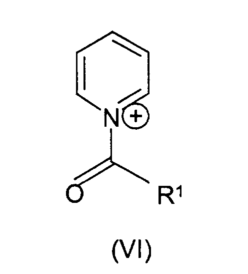 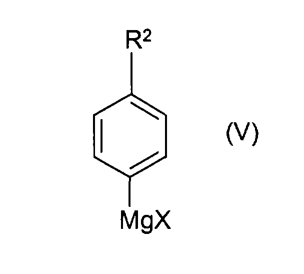 worin X die Bedeutung Cl oder Br hat.

9. Verfahren nach Anspruch 8, dadurch gekennzeichnet, dass der Cokatalysator ein Salz, ein Komplex oder eine metallische Form von Palladium oder Nickel ist.

10. Verfahren nach Anspruch 8 oder 9, dadurch gekennzeichnet, dass der Cokatalysator in einer Menge von 10⁻⁵ bis 10 mol%, vorzugsweise von 10⁻⁴ bis 7,5 mol%, bezogen auf die Grignard-Verbindung der Formel (V), eingesetzt wird.

11. Verfahren nach mindestens einem der Ansprüche 8 bis 10, dadurch gekennzeichnet, dass die Kupplung bei einer Temperatur von -70 bis +60°C, vorzugsweise von -50 bis +50°C, durchgeführt wird.

12. Verfahren nach mindestens einem der Ansprüche 8 bis 11, dadurch gekennzeichnet, dass die Cu-Verbindung Cul, CuBr, CuCI oder CuCl₂ ist.
